# DEMANDE DE BREVET EUROPEEN

(11) **EP 1 149 577 A1**
(43) Date de publication de la demande: **31.10.2001**
(21) Numéro de dépôt: 01400881.7
(22) Date de dépôt: 05.04.2001
(51) Int. Cl.: A61K 7/13

(54) **Composition pour la teinture d'oxydation des fibres kératiniques comprenant une 1-aminophenyl-pyrrolidine et un polymère cationique**

(30) Priorité: 18.04.2000 FR 0004993
(71) Demandeur: L'OREAL, 75008 Paris (FR)
(72) Inventeur: Kravtchenko, Sylvain, 92600 Asnieres (FR); Lagrange, Alain, 77700 Coupvray (FR)
(74) Mandataire: Miszputen, Laurent

(57) **Abrégé**

L'invention concerne une composition pour la teinture d'oxydation des fibres kératiniques, en particulier des fibres kératiniques humaines telles que les cheveux, comprenant, dans un milieu approprié pour la teinture, au moins une base d'oxydation choisie parmi les 1-(4-aminophényl)-pyrrolidines et leurs sels d'addition avec un acide, et au moins un polymère cationique particulier.

L'invention concerne également les procédés et dispositifs de teinture mettant en oeuvre ladite composition.

## Description

La présente invention concerne une composition pour la teinture d'oxydation des fibres kératiniques, en particulier des fibres kératiniques humaines telles que les cheveux, comprenant, dans un milieu approprié pour la teinture, au moins une base d'oxydation choisie parmi les 1-(4-aminophényl)-pyrrolidines et leurs sels d'addition avec un acide, et au moins polymère cationique particulier.

Il est connu de teindre les fibres kératiniques et en particulier les cheveux humains, avec des compositions de teinture contenant des précurseurs de colorants d'oxydation, généralement appelés "bases d'oxydation", en particulier des ortho- ou para- phénylènediamines, des ortho- ou para- aminophénols, et des bases hétérocycliques.

Les précurseurs de colorants d'oxydation sont des composés initialement peu ou pas colorés qui développent leur pouvoir tinctorial au sein du cheveu en présence d'agents oxydants en conduisant à la formation de composés colorés. La formation de ces composés colorés résulte, soit d'une condensation oxydative des "bases d'oxydation" sur elles-mêmes, soit d'une condensation oxydative des "bases d'oxydation" sur des composés modificateurs de coloration, ou "coupleurs", qui sont généralement présents dans les compositions tinctoriales utilisées en teinture d'oxydation et sont représentés plus particulièrement par des métaphénylènediamines, des méta-aminophénols et des métadiphénols, et certains composés hétérocycliques.

La variété des molécules mises en jeu, qui sont constituées d'une part par les "bases d'oxydation" et d'autre part par les "coupleurs", permet l'obtention d'une palette très riche en coloris.

La coloration dite "permanente" obtenue grâce à ces colorants d'oxydation, doit par ailleurs satisfaire un certain nombre d'exigences. Ainsi, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Ainsi, il est connu de longue date et d'un usage très répandu, de teindre les cheveux de façon permanente avec des produits de couplage de la paraphénylènediamine (PPD) en présence de peroxyde d'hydrogène. Cependant, de nouvelles bases d'oxydation mieux tolérées ont été recherchées et proposées comme alternatives à la PPD. Parmi elles, la base tertiaire N,N-bis(β-hydroxyéthyl)-paraphénylènediamine a été largement utilisée dans les produits de teinture capillaire du commerce.
Cependant, les colorations obtenues en mettant en oeuvre ces compositions ne sont pas toujours assez puissantes, chromatiques, ou résistantes aux différentes agressions que peuvent subir les cheveux.

Or, la Demanderesse vient maintenant de découvrir qu'il est possible d'obtenir de nouvelles teintures d'oxydation, capables de conduire à des colorations aux nuances variées, chromatiques, puissantes, esthétiques, peu sélectives, et résistant bien aux diverses agressions que peuvent subir les fibres, en associant au moins une base d'oxydation choisie parmi les 1-(4-aminophényl)-pyrrolidines et leurs sels d'addition avec un acide et au moins un polymère cationique particulier.

Cette découverte est à la base de la présente invention.

L'invention a donc pour premier objet une composition pour la teinture d'oxydation des fibres kératiniques et en particulier des fibres kératiniques humaines telles que les cheveux, comprenant dans un milieu approprié pour la teinture, **(i)** au moins une 1-(4-aminophényl)-pyrrolidine de formule (I) suivante à titre de précurseur de colorant d'oxydation : 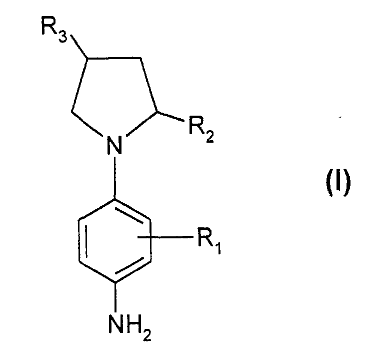 dans laquelle,
R₁ désigne un atome d'hydrogène, un radical alkyle en C₁-C₆, ou monohydroxyalkyle en C₁-C₅, ou polyhydroxyalkyle en C₂-C₅ ;
R₂ désigne un atome d'hydrogène, un radical -CONH₂, ou monohydroxyalkyle en C₁-C₅, ou polyhydroxyalkyle en C₂-C₅ ;
R₃ désigne un atome d'hydrogène, ou un radical OH ; et ses sels d'addition avec un acide,
**caractérisée** par le fait qu'elle comprend en outre au moins un polymère cationique choisi parmi :
**(1)** les homopolymères et copolymères comportant dans la chaîne des motifs de formule (II) suivante : 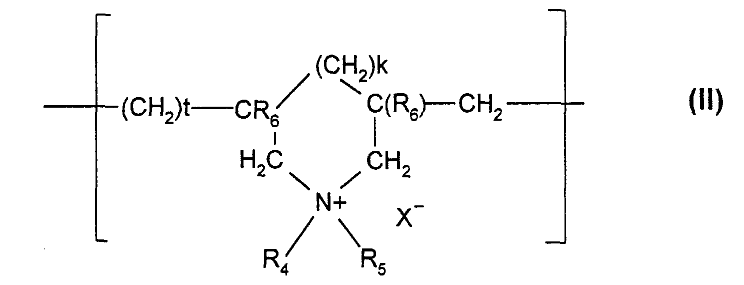 dans laquelle k et t sont égaux à 0 ou 1, la somme k + t étant égale à 1 ;
   R₄ et R₅, identiques ou différents, désignent un groupement alkyle ayant de 1 à 22 atomes de carbone, un groupement hydroxyalkyle(C₁-C₅), un groupement amidoalkyle(C₁-C₄), ou R₄ et R₅ désignent conjointement avec l'atome d'azote auquel ils sont rattachés, un groupement pipéridinyle ou morpholinyle ;
   R₆ désigne un atome d'hydrogène ou un radical méthyle ;
   X⁻ est un anion;
**(2)** les polymères de diammonium quaternaire contenant des motifs récurrents de formule (III) suivante : 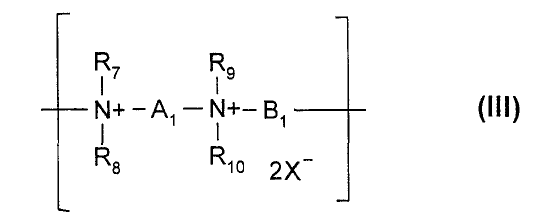 dans laquelle :
   R₇, R₈, R₉ et R₁₀, identiques ou différents, représentent des radicaux aliphatiques, alicycliques, ou arylaliphatiques contenant de 1 à 20 atomes de carbone ou des radicaux hydroxyalkylaliphatiques inférieurs,
      ou bien R₇, R₈, R₉ et R₁₀, ensemble ou séparément, constituent avec les atomes d'azote auxquels ils sont rattachés des hétérocycles contenant éventuellement un second hétéroatome autre que l'azote,
      ou bien R₇, R₈, Rg et R₁₀, représentent un radical alkyle en C₁-C₆ linéaire ou ramifié substitué par un groupement nitrile, ester, acyle, amide, ou -CO-O- R₁₁-D, ou -CO-NH-R₁₁-D dans lesquels R₁₁ est un alkylène et D un groupement ammonium quaternaire ;
   A₁ et B₁ représentent des groupements polyméthyléniques contenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
   X⁻ est un anion;
   A₁, R₇ et R₉ peuvent former avec les deux atomes d'azote auxquels ils sont rattachés, un cycle pipérazinique ; en outre si A₁ désigne un radical alkylène ou hydroxyalkylène linéaire ou ramifié, saturé ou insaturé, B₁ peut également désigner un groupement -(CH₂)n-CO-D-OC-(CH₂)n- dans lequel n est compris entre 1 et 100 et de préférence entre 1 et 50, et D désigne :
      a) un reste de glycol de formule : -O-Z-O-, où Z désigne un radical hydrocarboné linéaire ou ramifié ou un groupement répondant à l'une des formules suivantes :

         -(CH₂-CH₂-O)x-CH₂-CH₂-

         -[CH₂-CH(CH₃)-O]y-CH₂-CH(CH₃)-

         où x et y désignent un nombre entier de 1 à 4, représentant un degré de polymérisation défini et unique ou un nombre quelconque de 1 à 4 représentant un degré de polymérisation moyen ;
      b) un reste de diamine bis-secondaire tel qu'un dérivé de pipérazine ;
      c) un reste de diamine bis-primaire de formule : -NH-Y-NH-, où Y désigne un radical hydrocarboné linéaire ou ramifié, ou bien le radical bivalent

         -CH₂-CH₂-S-S-CH₂-CH₂- ;
      d) un groupement uréylène de formule : -NH-CO-NH- ;
**(3)** les polymères de diammonium quaternaire constitués de motifs de formule (IV) suivante: 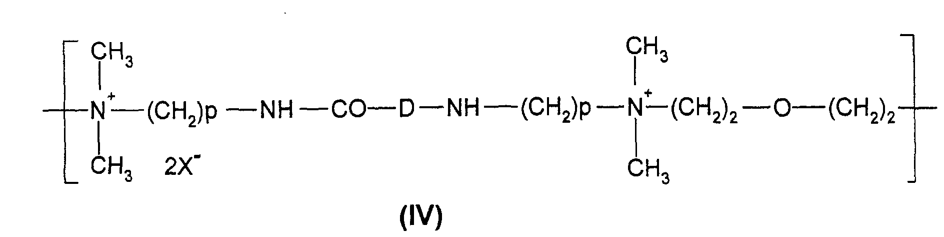 dans laquelle :
   p désigne un nombre entier variant de 1 à 6 environ,
   D peut être nul ou peut représenter un groupement ―(CH₂)ᵣ―CO― dans lequel r désigne un nombre égal à 4 ou à 7, et
   X⁻ est un anion
(**4**) les silicones aminées.

Les sels d'addition avec un acide des 1-(4-aminophényl)-pyrrolidines de formule (I) utilisables dans les compositions tinctoriales selon l'invention sont notamment choisis parmi les chlorhydrates, les bromhydrates, les sulfates, les tartrates, les lactates et les acétates.

La composition tinctoriale conforme à l'invention ainsi définie, conduit après mélange avec une composition oxydante, à des colorations dans des nuances variées, chromatiques, puissantes, esthétiques, présentant une faible sélectivité et d'excellentes propriétés de résistances à la fois vis-à-vis des agents atmosphériques tels que la lumière et les intempéries et vis-à-vis de la transpiration et des différents traitements que peuvent subir les cheveux.

Un autre objet de l'invention porte sur une composition prête à l'emploi pour la teinture d'oxydation des fibres kératiniques qui comprend, dans un milieu approprié pour la teinture, au moins une 1-(4-aminophényl)-pyrrolidine de formule (I), au moins un polymère cationique particulier et au moins un agent oxydant.
Par composition prête à l'emploi, on entend au sens de la présente invention, toute composition destinée à être appliquée immédiatement sur les fibres kératiniques.

L'invention vise également un procédé de teinture d'oxydation des fibres kératiniques, et en particulier des fibres kératiniques humaines telles que les cheveux, consistant à appliquer sur les fibres une composition colorante contenant dans un milieu approprié pour la teinture, au moins une 1-(4-aminophényl)-pyrrolidine de formule (I), la couleur étant révélée à pH alcalin, neutre ou acide à l'aide d'une composition oxydante qui est mélangée juste au moment de l'emploi à la composition colorante ou qui est appliquée séquentiellement sans rinçage intermédiaire, au moins un polymère cationique particulier étant présent dans la composition colorante et/ou oxydante.

L'invention a également pour objet des dispositifs de teinture à plusieurs compartiments ou " kits " pour la teinture d'oxydation des fibres kératiniques, en particulier des fibres kératiniques humaines telles que les cheveux.
De tels dispositifs comportent un premier compartiment contenant au moins une 1-(4-aminophényl)-pyrrolidine de formule (I) et un deuxième compartiment contenant au moins un agent oxydant, au moins un polymère cationique particulier étant présent dans le premier compartiment et/ou dans le second compartiment.
Un autre dispositif de teinture à plusieurs compartiments comporte au moins un compartiment contenant au moins une 1-(4-aminophényl)-pyrrolidine de formule (I), au moins un compartiment contenant au moins un polymère cationique particulier, et au moins un autre compartiment contenant au moins un agent oxydant.

Mais d'autres caractéristiques, aspects, objets et avantages de l'invention apparaîtront encore plus clairement à la lecture de la description et des exemples qui suivent.

Le ou les 1-(4-aminophényl)-pyrrolidines de formule (I) selon l'invention sont des composés bien connus de l'homme de l'art et notamment décrits et préparés dans les brevets américains N°- 5851237, 5876464, et 5993491.

Selon la présente invention, on préfère tout particulièrement utiliser des 1-(4-aminophényl)-pyrrolidines de formule (I) pour laquelle :
- R₁, R₂ et R₃ désignent un atome d'hydrogène; le composé de formule (I) est alors la 1-(4-aminophényl)-pyrrolidine, ou,
- R₁ et R₃ désignent un atome d'hydrogène et R₂ désigne le radical -CH₂OH; le composé de formule (I) est alors le 1-(4-aminophényl)-2-pyrrolidineméthanol, ou,
- R₁ désigne un atome d'hydrogène, R₂ désigne le radical -CH₂OH et R₃ désigne le radical OH; le composé de formule (I) est alors le 1-(4-aminophényl)-4-hydroxy-2-pyrrolidineméthanol, ou,
- R₁ et R₃ désignent un atome d'hydrogène et R₂ désigne le radical -CONH₂; le composé de formule (I) est alors la N-(4-aminophényl)-prolineamide.

Le ou les 1-(4-aminophényl)-pyrrolidines de formule (I) utilisés conformément à l'invention représentent de préférence de 0,001 à 10 % en poids environ du poids total de la composition conforme à l'invention et encore plus préférentiellement de 0,01 à 8% environ de ce poids.

Les polymères cationiques utilisables selon l'invention ont généralement une masse moléculaire moyenne en nombre comprise entre 500 et 5.10⁶ environ, et de préférence comprise entre 10³ et 3.10⁶ environ.

De préférence selon la présente invention, dans les polymères cationiques comportant des motifs de formule (II), R₇ et R₈ identiques ou différents désignent un groupement alkyle ayant de 1 à 4 atomes de carbone ; X⁻ est un anion bromure, chlorure, acétate, borate, citrate, tartrate, bisulfate, bisulfite, sulfate, phosphate. De tels polymères sont notamment décrits dans le brevet français 2.080.759 et dans son certificat d'addition 2.190.406.
On peut citer plus particulièrement parmi eux, l'homopolymère de chlorure de diméthyldiallylammonium vendu sous la dénomination "Merquat 100" par la société Calgon (et ses homologues de faible masse moléculaire moyenne en poids) et les copolymères de chlorure de diméthyldiallylammonium et d'acrylamide commercialisés sous la dénomination "MERQUAT 550".

Les polymères cationiques comportant des motifs de formule (III) ont une masse moléculaire moyenne en nombre généralement comprise entre 1000 et 100000.
Dans ces polymères, X⁻ est un anion chlorure ou bromure.
Des polymères de ce type sont notamment décrits dans les brevets français 2.320.330, 2.270.846, 2.316.271, 2.336.434 et 2.413.907 et les brevets US 2.273.780, 2.375.853, 2.388.614, 2.454.547, 3.206.462, 2.261.002, 2.271.378, 3.874.870, 4.001.432, 3.929.990, 3.966.904, 4.005.193, 4.025.617, 4.025.627, 4.025.653, 4.026.945 et 4.027.020.

On peut utiliser plus particulièrement parmi eux, les polymères qui sont constitués de motifs récurrents répondant à la formule (V) suivante: 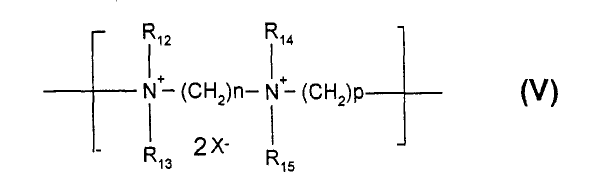 dans laquelle R₁₂, R₁₃, R₁₄ et R₁₅, identiques ou différents, désignent un radical alkyle ou hydroxyalkyle ayant de 1 à 4 atomes de carbone environ, n et p sont des nombres entiers variant de 2 à 20.
Encore plus particulièrement, on préfère les composés de formule (V) dans laquelle R₁₂, R₁₃, R₁₄ et R₁₅ représentent un radical méthyle ou éthyle.
Des polymères cationiques de formule (V) particulièrement préférés sont ceux pour lesquels R₁₂, R₁₃, R₁₄ et R₁₅ représentent un radical méthyle et n = 3, p = 6 et X = Cl, et notamment ceux dont le poids moléculaire, déterminé par chromatographie par perméation de gel, est compris entre 9500 et 9900 [Polymère W].
D'autres polymères cationiques de formule (V) particulièrement préférés sont ceux pour lesquels R₁₂ et R₁₃ représentent un radical méthyle, R₁₄ et R₁₅ représentent un radical éthyle et n = p = 3 et X = Br, et notamment ceux dont le poids moléculaire, déterminé par chromatographie par perméation de gel, est d'environ 1200 [Polymère U].

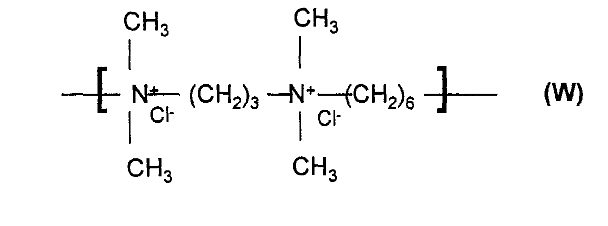

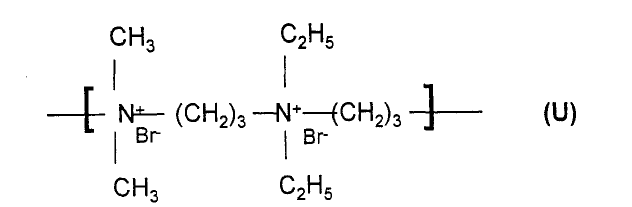

Lesdits polymères à motifs (W) et (U) sont préparés et décrits dans le brevet français 2 270 846.

Les polymères cationiques comportant des motifs de formule (IV) sont notamment décrits dans la demande de brevet EP-A-122 324.
Parmi ces polymères, on préfère ceux de masse moléculaire mesurée par RMN du Carbone 13 inférieure à 100000, et dans la formule de laquelle :
p désigne un nombre entier variant de 1 à 6 environ,
D peut être nul ou peut représenter un groupement ―(CH₂)ᵣ―CO― dans lequel r désigne un nombre égal à 4 ou à 7,
et X⁻ est un anion dérivé d'un acide minéral ou organique.

Parmi lesdits polymères à motifs de formule (IV), on préfère encore ceux pour lesquels, p est égal à 3, et,
a) D représente un groupement ―(CH₂)₄―CO―, X désigne un atome de chlore, la masse moléculaire mesurée par RMN du Carbone 13 ( RMN¹³C) étant d'environ 5600 ; un polymère de ce type est proposé par la société MIRANOL sous le nom de MIRAPOL-AD1,
b) D représente un groupement ―(CH₂)₇―CO―, X désigne un atome de chlore, la masse moléculaire mesurée par RMN du Carbone 13 ( RMN¹³C) étant d'environ 8100 ; un polymère de ce type est proposé par la société MIRANOL sous le nom de MIRAPOL-AZ1,
c) D désigne la valeur zéro, X désigne un atome de chlore, la masse moléculaire mesurée par RMN du Carbone 13, ( RMN¹³C ) étant d'environ 25500 ; un polymère de ce type est vendu par la société MIRANOL sous le nom MIRAPOL-A15,
d) un " Block Copolymer " formé de motifs correspondant aux polymères décrits aux alinéas a) et c), proposé par la société MIRANOL sous les noms MIRAPOL-9, (masse moléculaire RMN¹³C, environ 7800) MIRAPOL-175, (masse moléculaire RMN¹³C, environ 8000) MIRAPOL-95, (masse moléculaire RMN¹³C, environ 12500).
   Plus particulièrement encore, on préfère selon l'invention le polymère à motifs de formule (IV) dans laquelle p est égal à 3, D désigne la valeur zéro, X désigne un atome de chlore, la masse moléculaire mesurée par RMN du Carbone 13, (RMN¹³C) étant d'environ 25500.
   Lesdits polymères cationiques à motifs de formule (IV) peuvent être préparés selon les procédés décrits dans les brevets U.S.A. n° 4 157 388, 4 390 689, 4 702 906,4 719 282.

Selon l'invention, on désigne par silicone aminée toute silicone comportant au moins une amine primaire, secondaire, tertiaire ou un groupement ammonium quaternaire .
Conformément à l'invention, les silicones aminées sont choisies parmi :
**(i)** les composés dénommés dans le dictionnaire CTFA, "amodiméthicone" et répondant à la formule (VI) suivante : 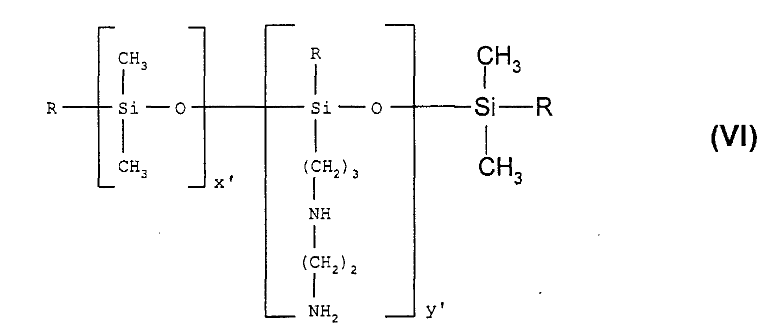 dans laquelle R désigne le radical CH₃ ou OH, et
   x' et y' sont des nombres entiers dépendant du poids moléculaire, généralement tels que ledit poids moléculaire en nombre est compris entre 5 000 et 500 000 environ ;
**(ii)** les composés répondant à la formule (VII) suivante :

   (R¹)ₐ (T)₃₋ₐ―Si**[**OSi(T)₂**]**ₙ―**[**OSi(T)_{b}(R¹)_{2-b}**]**ₘ―OSi(T)₃₋ₐ― (R¹)ₐ (VII)

   dans laquelle,
   T est un atome d'hydrogène, ou un radical phényle, ou OH, ou alkyle en C₁-C₈, et de préférence méthyle,
   a désigne le nombre 0 ou un nombre entier de 1 à 3, et de préférence 0,
   b désigne 0 ou 1, et en particulier 1,
   m et n sont des nombres tels que la somme (n + m) peut varier notamment de 1 à 2 000 et en particulier de 50 à 150, n pouvant désigner un nombre de 0 à 1 999 et notamment de 49 à 149 et m pouvant désigner un nombre de 1 à 2 000, et notamment de 1 à 10 ;
   R¹ est un radical monovalent de formule -C_{q}H_{2q}L dans laquelle q est un nombre de 2 à 8 et L est un groupement aminé éventuellement quaternisé choisi parmi les groupements :
      -N(R²)-CH₂-CH₂-N(R²)₂
      -N(R²)₂
      -N^{⊕}(R²)₃Q⁻
      -N^{⊕}(R²)(H)₂Q⁻
      -N^{⊕}(R²)₂H Q⁻
      -N(R²)-CH₂-CH₂-N^{⊕}(R²)(H)₂ Q⁻,
      dans lesquels R² peut désigner hydrogène, phényle, benzyle, ou un radical hydrocarboné saturé monovalent, par exemple un radical alkyle ayant de 1 à 20 atomes de carbone et Q⁻ représente un ion halogénure tel que par exemple fluorure, chlorure, bromure ou iodure.
      Un produit correspondant à cette définition est le polymère dénommé dans le dictionnaire CTFA "triméthylsilylamodiméthicone", répondant à la formule (VIII) suivante 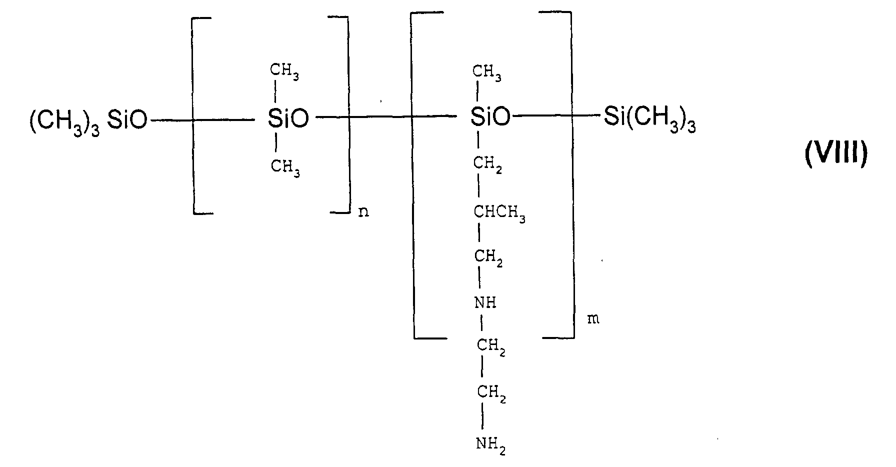 dans laquelle n et m ont les significations données ci-dessus [cf formule (VII)]. De tels composés sont décrits par exemple dans la demande de brevet EP-A-95238; un composé de formule (VIII) est par exemple vendu sous la dénomination Q2-8220 par la société OSI.
**(iii)** les composés répondant à la formule (IX) suivante : 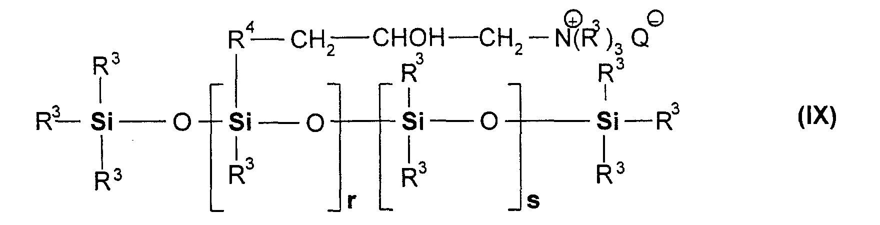 dans laquelle,
   R³ représente un radical hydrocarboné monovalent ayant de 1 à 18 atomes de carbone, et en particulier un radical alkyle en C₁-C₁₈, ou alcényle en C₂-C₁₈,
   R⁴ représente un radical hydrocarboné divalent, notamment un radical alkylène en C₁-C₁₈ ou un radical alkylèneoxy divalent en C₁-C₁₈, et par exemple en C₁-C₈ ;
   Q⁻ est un ion halogénure, notamment chlorure ;
   r représente une valeur statistique moyenne de 2 à 20 et en particulier de 2 à 8 ;
   s représente une valeur statistique moyenne de 20 à 200 et en particulier de 20 à 50.

De tels composés sont décrits plus particulièrement dans le brevet US-4 185 087. Un composé entrant dans cette classe est celui vendu par la Société Union Carbide sous la dénomination "Ucar Silicone ALE 56".

Lorsque ces silicones aminées sont mises en oeuvre, une forme de réalisation particulièrement intéressante est leur utilisation conjointe avec des agents de surface cationiques et/ou non ioniques.

A titre d'exemple, on peut utiliser le produit vendu sous la dénomination "Emulsion Cationique DC 939" par la Société Dow Corning, qui comprend, outre l'amodiméthicone, un agent de surface cationique qui est le chlorure de triméthylcétylammonium et un agent de surface non ionique de formule : C₁₃H₂₇-(OC₂H₄)₁₂-OH, connu sous la dénomination CTFA "tridéceth-12".

Un autre produit commercial utilisable selon l'invention est le produit vendu sous la dénomination "Dow Corning Q2 7224" par la Société Dow Corning, comportant en association le triméthylsilylamodiméthicone de formule (VIII) décrite ci-dessus, un agent de surface non ionique de formule : C₈H₁₇-C₆H₄-(OCH₂CH₂)₄₀-OH, connu sous la dénomination CTFA "octoxynol-40", un second agent de surface non ionique de formule: C₁₂H₂₅-(OCH₂-CH₂)₆-OH, connu sous la dénomination CTFA "isolaureth-6", et du propylèneglycol.

**(iv)** les composés de formule (X) suivante: 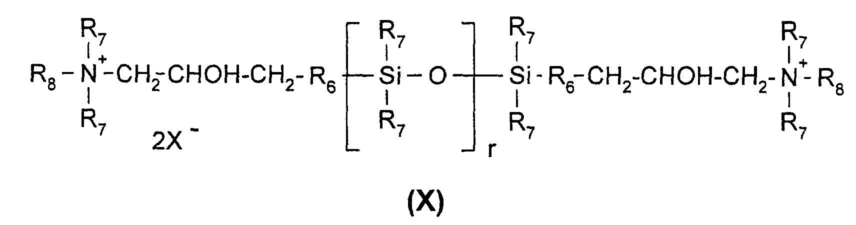 dans laquelle :
R₆ représente un radical hydrocarboné divalent, notamment un radical alkylène en C₁-C₁₈ ou un radical alkylèneoxy divalent en C₁-C₁₈ relié au Si par une liaison SiC;
les radicaux R₇, identiques ou différents, représentent un radical hydrocarboné monovalent ayant de 1 à 18 atomes de carbone, un radical alcényle en C₂-C₁₈ ou un cycle comprenant 5 ou 6 atomes de carbone ;
les radicaux R₈, identiques ou différents, représentent un atome d'hydrogène, un radical hydrocarboné monovalent ayant de 1 à 18 atomes de carbone, un radical alcényle en C₂-C₁₈ , un radical -R₆-NHCOR₇ ;
r représente une valeur statistique moyenne de 2 à 200 ;
X- est un anion tel qu'un ion halogénure, notamment chlorure, ou un sel d'acide organique (acétate ...);
Les silicones cationiques de formule (X) sont par exemple décrites dans la demande EP-A-0530974.
Des silicones entrant dans cette classe sont les silicones commercialisées par la société GOLDSCHMIDT sous les dénominations ABIL QUAT 3270, ABIL QUAT 3272, ABIL QUAT 3474.

La concentration en polymère cationique dans les compositions selon la présente invention peut varier de 0,01 à 10% en poids par rapport au poids total de la composition, de préférence de 0,05 à 5% et plus préférentiellement encore de 0,1 à 3%.

De préférence, les compositions de l'invention contiennent au moins un coupleur. Parmi ces coupleurs, on peut notamment citer les métaphénylènediamines, les méta-aminophénols, les métadiphénols, les naphtols et les coupleurs hétérocycliques tels que par exemple les dérivés indoliques, les dérivés indoliniques, le sésamol et ses dérivés, les dérivés pyridiniques, les dérivés pyrazolotriazoles, les pyrazolones, les indazoles, les benzimidazoles, les benzothiazoles, les benzoxazoles, les 1,3-benzodioxoles, les quinolines et leurs sels d'addition avec un acide.

Ces coupleurs sont plus particulièrement choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

Le ou les coupleurs peuvent être présents dans la dite composition selon l'invention à une concentration comprise entre 0,0001 et 15% en poids par rapport au poids total de la composition.

La composition tinctoriale conforme à l'invention peut, en outre, renfermer au moins une base d'oxydation additionnelle différente des 1-(4-aminophényl)-pyrrolidines de formule (I) et/ou au moins un colorant direct .
Parmi les bases d'oxydation additionnelles utilisables selon l'invention, on peut citer la paraphénylènediamine, la paratoluylènediamine, la 2-hydroxyéthylparaphénylènediamine, la 1-N,N-bis(2-hydroxyéthyl)-paraphénylènediamine, les para-aminophénols tels que le 3-méthyl-4-aminophénol et le 4-aminophénol, les orthophénylènes diamines, les orthoaminophénols, les bases doubles, les bases hétérocycliques comme les pyrimidines telles que la 2,4,5,6-tétraaminopyrimidine ou comme les pyrazoles tel que le 1-(2-hydroxyéthyl)-4,5-diamino-pyrazole, et leurs sels d'addition avec un acide.
La ou les bases d'oxydation additionnelles peuvent être présentes à une concentration comprise entre 0,0001 et 15% en poids par rapport au poids total de ladite composition.
Selon une forme de réalisation préférée, la composition tinctoriale conforme à l'invention peut en outre renfermer un ou plusieurs colorants directs notamment pour modifier les nuances en les enrichissant de reflets. Ces colorants directs peuvent alors être choisis parmi les colorants nitrés, azoïques ou anthraquinoniques, neutres, cationiques ou anioniques dans la proportion pondérale d'environ 0,001 à 20% et de préférence de 0,01 à 10% du poids total de la composition.

Le milieu de la composition approprié pour la teinture est de préférence un milieu aqueux constitué par de l'eau et peut avantageusement contenir des solvants organiques acceptables sur le plan cosmétique, dont plus particulièrement, des alcools tels que l'alcool éthylique, l'alcool isopropylique, l'alcool benzylique, et l'alcool phényléthylique, ou des glycols ou éthers de glycol tels que, par exemple, les éthers monométhylique, monoéthylique et monobutylique d'éthylèneglycol, le propylèneglycol ou ses éthers tels que, par exemple, le monométhyléther de propylèneglycol, le butylèneglycol, le dipropylèneglycol ainsi que les alkyléthers de diéthylèneglycol comme par exemple, le monoéthyléther ou le monobutyléther du diéthylèneglycol, dans des concentrations comprises entre environ 0,5 et 20% et, de préférence, entre environ 2 et 10% en poids par rapport au poids total de la composition.

La composition selon l'invention peut encore contenir une quantité efficace d'autres agents, par ailleurs antérieurement connus en coloration d'oxydation, tels que divers adjuvants usuels comme des séquestrants tel que l'EDTA et l'acide étidronique, des filtres UV, des cires, des silicones volatiles ou non, cycliques ou linéaires ou ramifiées, des conservateurs, des céramides, des pseudocéramides, des huiles végétales, minérales ou de synthèse, les vitamines ou provitamines comme le panthénol, des opacifiants, des agents épaississants tels que les acides polyacryliques réticulés ou les hydroxyalkylcelluloses etc....

Ladite composition peut également contenir des agents réducteurs ou antioxydants. Ceux-ci peuvent être choisis en particulier parmi le sulfite de sodium, l'acide thioglycolique, l'acide thiolactique, le bisulfite de sodium, l'acide déhydroascorbique, l'hydroquinone, la 2-méthyl-hydroquinone, la ter-butylhydroquinone et l'acide homogentisique, et ils sont alors généralement présents dans des quantités allant d'environ 0,05 à 1,5% en poids par rapport au poids total de la composition.

La composition selon l'invention peut également contenir un ou plusieurs alcools gras, ces alcools gras étant introduits sous forme pure ou de mélange. On peut citer parmi eux plus particulièrement les alcools laurique, cétylique, stéarylique, oléique et leurs mélanges. Ces alcools gras peuvent représenter de 0,001 à 20% en poids environ du poids total de la composition.

De préférence, la composition colorante et/ou la composition oxydante de la composition prête à l'emploi selon l'invention contient au moins un tensioactif nonionique, anionique, cationique ou amphotère dans la proportion d'environ 0,1 à 20% en poids.
Encore plus préférentiellement ladite composition contient au moins un tensioactif nonionique.

Bien entendu, l'homme de l'art veillera à choisir le ou les éventuels composés complémentaires mentionnés ci-avant, de manière telle que les propriétés avantageuses attachées intrinsèquement à la composition de teinture d'oxydation selon l'invention ne soient pas, ou substantiellement pas, altérées par la ou les adjonctions envisagées.

Dans la composition oxydante, l'agent oxydant est choisi de préférence parmi le peroxyde d'hydrogène, le peroxyde d'urée, les bromates ou ferricyanures de métaux alcalins, les persels tels que les perborates et les persulfates. L'utilisation du peroxyde d'hydrogène est particulièrement préférée. Cet agent oxydant est avantageusement constitué par une solution d'eau oxygénée dont le titre peut varier, plus particulièrement, d'environ 1 à 40 volumes, et encore plus préférentiellement d'environ 5 à 40.
On peut également utiliser à titre d'agent oxydant une ou plusieurs enzymes d'oxydoréduction telles que les laccases, les peroxydases et les oxydoréductases à 2 électrons (telles que l'uricase), le cas échéant en présence de leur donneur ou cofacteur respectif.

Le pH de la composition colorante ou de la composition prête à l'emploi et appliquée sur les fibres kératiniques [composition résultant du mélange de la composition colorante selon l'invention et de la composition oxydante], est généralement compris entre les valeurs 3 et 12. Il est de préférence compris entre 6 et 11, et peut être ajusté à la valeur désirée au moyen d'agents acidifiants ou alcalinisants bien connus de l'état de la technique en teinture des fibres kératiniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxyalkylamines et les ethylènediamines oxyéthylénées et/ou oxypropylénées, les hydroxydes de sodium ou de potassium et les composés de formule (XI) suivante : 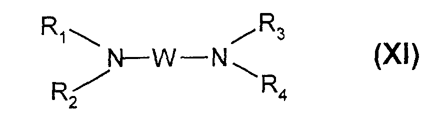 dans laquelle, W est un reste propylène éventuellement substitué par un groupement hydroxyle ou un radical alkyle en C₁-C₄ ; R₁, R₂, R₃ et R₄, identiques ou différents, représentent un atome d'hydrogène, un radical alkyle en C₁-C₄ ou hydroxyalkyle en C₁-C₄.

Les agents acidifiants sont classiquement, à titre d'exemple, des acides minéraux ou organiques comme l'acide chlorhydrique, l'acide orthophosphorique, des acides carboxyliques comme l'acide tartrique, l'acide citrique, l'acide lactique, ou des acides sulfoniques.

La composition tinctoriale conforme à l'invention peut se présenter sous des formes diverses, telles que sous forme de liquides, de poudres, de crèmes, de gels, éventuellement pressurisés, ou sous toute autre forme appropriée pour réaliser une teinture des fibres kératiniques, et notamment des cheveux humains.

Le procédé de teinture selon l'invention consiste, de préférence, à appliquer la composition prête à l'emploi, réalisée extemporanément au moment de l'emploi à partir de la composition colorante selon l'invention et de la composition oxydante décrites ci-avant, sur les fibres kératiniques sèches ou humides, et à la laisser agir pendant un temps de pause variant, de préférence, de 1 à 60 minutes environ, et plus préférentiellement de 10 à 45 minutes environ, à rincer les fibres, puis éventuellement à les laver au shampooing, puis à les rincer à nouveau, et à les sécher.

Les exemples qui suivent sont destinés à illustrer l'invention.

### EXEMPLES

On a préparé les compositions tinctoriales suivantes : (exprimées en grammes)

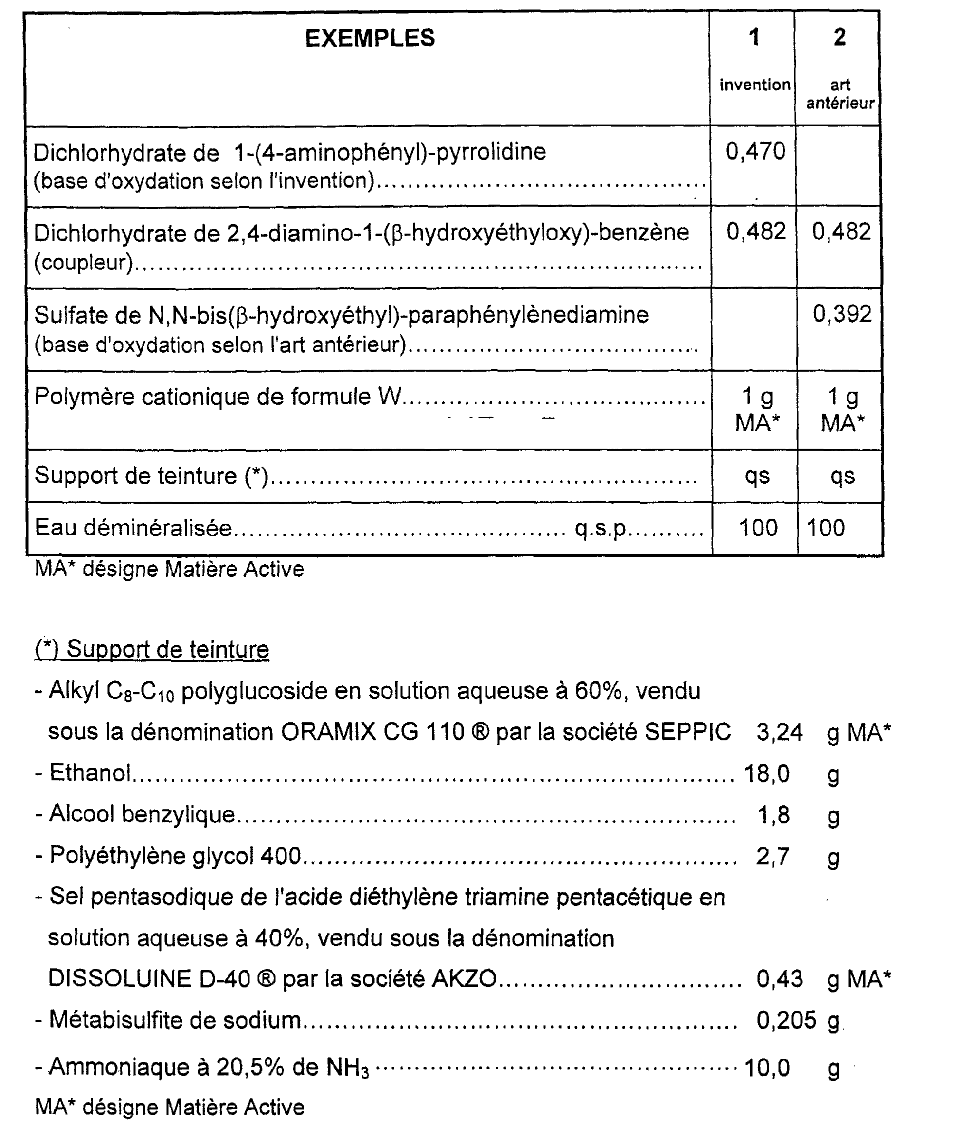

Au moment de l'emploi, on a mélangé poids pour poids chacune des compositions tinctoriales décrites ci-dessus avec une solution de peroxyde d'hydrogène à 20 volumes (6% en poids).

Les mélanges ainsi réalisés ont été appliqués pendant 30 minutes, sur des mèches de cheveux gris naturels à 90 % de blancs. Les mèches ont ensuite été rincées, lavées avec un shampooing standard, rincées à nouveau puis séchées.

La couleur a ensuite été mesurée au colorimètre MINOLTA CM2002 dans le système L* a* b*.
Dans le système L* a* b* les 3 paramètres désignent respectivement l'intensité (L*), la nuance (a*), et la saturation (b*).
Selon ce système, plus la valeur de L est élevée, plus la couleur est claire ou peu intense. Inversement, plus la valeur de L est faible, plus la couleur est foncée ou très intense.

Les résultats ont été réunis dans le tableau (I) ci-dessous.

**Tableau (I)**

| EXEMPLES | L* |
|---|---|
| 2 | 25.91 |
| 1 | 22.27 |

### Conclusion:

La teinture avec l'association selon l'invention (1) est plus puissante que celle de l'art antérieur (2) [valeur de L plus basse].

## Revendications

1. Composition pour la teinture d'oxydation des fibres kératiniques, en particulier des fibres kératiniques humaines telles que les cheveux, comprenant, dans un milieu approprié pour la teinture, (i) au moins une 1-(4-aminophényl)-pyrrolidine de formule (I) suivante à titre de précurseur de colorant d'oxydation : 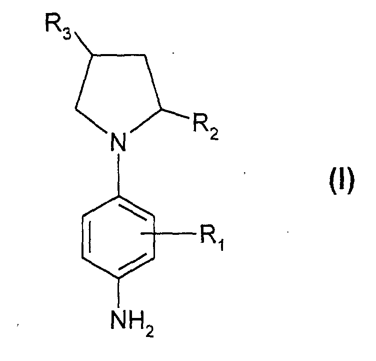 dans laquelle,
R₁ désigne un atome d'hydrogène, un radical alkyle en C₁-C₆, ou monohydroxyalkyle en C₁-C₅, ou polyhydroxyalkyle en C₂-C₅ ;
R₂ désigne un atome d'hydrogène, un radical -CONH₂, ou monohydroxyalkyle en C₁-C₅, ou polyhydroxyalkyle en C₂-C₅ ;
R₃ désigne un atome d'hydrogène, ou un radical OH ;
et ses sels d'addition avec un acide,
**caractérisée par le fait qu'**elle comprend en outre au moins un polymère cationique choisi parmi :
-**(1)** les homopolymères et copolymères comportant dans la chaîne des motifs de formule (II) suivante : 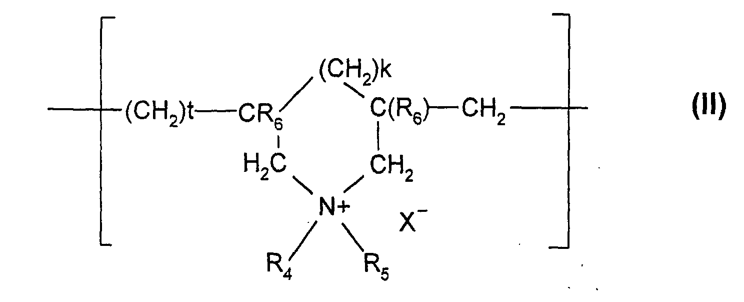
dans laquelle k et t sont égaux à 0 ou 1, la somme k + t étant égale à 1 ;
R₄ et R₅, identiques ou différents, désignent un groupement alkyle ayant de 1 à 22 atomes de carbone, un groupement hydroxyalkyle(C₁-C₅), un groupement amidoalkyle(C₁-C₄), ou R₄ et R₅ désignent conjointement avec l'atome d'azote auquel ils sont rattachés, un groupement pipéridinyle ou morpholinyle ;
R₆ désigne un atome d'hydrogène ou un radical méthyle ;
X⁻ est un anion;
-(**2**) les polymères de diammonium quaternaire contenant des motifs récurrents de formule (III) suivante : 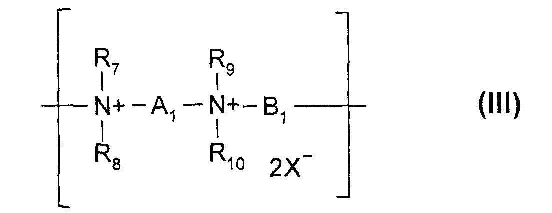 dans laquelle :
R₇, R₈, R₉ et R₁₀, identiques ou différents, représentent des radicaux aliphatiques, alicycliques, ou arylaliphatiques contenant de 1 à 20 atomes de carbone ou des radicaux hydroxyalkylaliphatiques inférieurs, ou bien R₇, R₈, R₉ et R₁₀, ensemble ou séparément, constituent avec les atomes d'azote auxquels ils sont rattachés des hétérocycles contenant éventuellement un second hétéroatome autre que l'azote, ou bien R₇, R₈, R₉ et R₁₀, représentent un radical alkyle en C₁-C₆ linéaire ou ramifié substitué par un groupement nitrile, ester, acyle, amide, ou -CO-O-R₁₁-D, ou -CO-NH-R₁₁-D dans lesquels R₁₁ est un alkylène et D un groupement ammonium quaternaire ;
A₁ et B₁ représentent des groupements polyméthyléniques contenant de 2 à 20 atomes de carbone, linéaires ou ramifiés, saturés ou insaturés, et pouvant contenir, liés à ou intercalés dans la chaîne principale, un ou plusieurs cycles aromatiques, ou un ou plusieurs atomes d'oxygène, de soufre ou des groupements sulfoxyde, sulfone, disulfure, amino, alkylamino, hydroxyle, ammonium quaternaire, uréido, amide ou ester, et
X⁻ est un anion;
A₁, R₇ et R₉ peuvent former avec les deux atomes d'azote auxquels ils sont rattachés, un cycle pipérazinique ; en outre si A₁ désigne un radical alkylène ou hydroxyalkylène linéaire ou ramifié, saturé ou insaturé, B₁ peut également désigner un groupement -(CH₂)n-CO-D-OC-(CH₂)n- dans lequel n est compris entre 1 et 100 et de préférence entre 1 et 50, et D désigne :
a) un reste de glycol de formule : -O-Z-O-, où Z désigne un radical hydrocarboné linéaire ou ramifié ou un groupement répondant à l'une des formules suivantes :
-(CH₂-CH₂-O)x-CH₂-CH₂-
-[CH₂-CH(CH₃)-O]y-CH₂-CH(CH₃)-
où x et y désignent un nombre entier de 1 à 4, représentant un degré de polymérisation défini et unique ou un nombre quelconque de 1 à 4 représentant un degré de polymérisation moyen ;
b) un reste de diamine bis-secondaire tel qu'un dérivé de pipérazine ;
c) un reste de diamine bis-primaire de formule : -NH-Y-NH-, où Y désigne un radical hydrocarboné linéaire ou ramifié, ou bien le radical bivalent
-CH₂-CH₂-S-S-CH₂-CH₂- ;
d) un groupement uréylène de formule : -NH-CO-NH- ;
- **(3)** les polymères de diammonium quaternaire constitués de motifs de formule (IV) suivante: 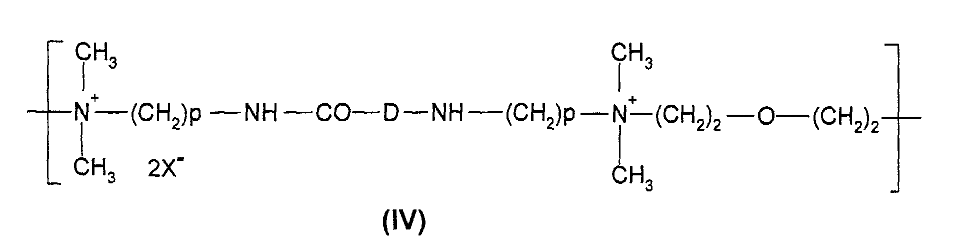 dans laquelle :
p désigne un nombre entier variant de 1 à 6,
D est nul ou représente un groupement ―(CH₂)ᵣ―CO― dans lequel r désigne un nombre égal à 4 ou à 7, et
X⁻ est un anion .
-(**4**) les silicones aminées.

2. Composition selon la revendication 1, **caractérisée par le fait que** dans la formule (I) des 1-(4-aminophényl)-pyrrolidines, R₁, R₂ et R₃ désignent un atome d'hydrogène.

3. Composition selon la revendication 1, **caractérisée par le fait que** dans la formule (I) des 1-(4-aminophényl)-pyrrolidines, R₁ et R₃ désignent un atome d'hydrogène et R₂ désigne le radical -CH₂OH.

4. Composition selon la revendication 1, **caractérisée par le fait que** dans la formule (I) des 1-(4-aminophényl)-pyrrolidines, R₁ désigne un atome d'hydrogène, R₂ désigne le radical -CH₂OH, et R₃ désigne le radical -OH.

5. Composition selon la revendication 1, **caractérisée par le fait que** dans la formule (I) des 1-(4-aminophényl)-pyrrolidines, R₁ et R₃ désignent un atome d'hydrogène, et R₂ désigne le radical -CONH₂.

6. Composition selon l'une quelconque des revendications précédentes, **caractérisée par le fait que** les sels d'addition avec un acide des 1-(4-aminophényl)-pyrrolidines de formule (I) sont choisis parmi les chlorhydrates, les bromhydrates, les sulfates, les tartrates, les lactates et les acétates.

7. Composition selon l'une quelconque des revendications précédentes,
**caractérisée par le fait que** les 1-(4-aminophényl)-pyrrolidines de formule (I) et leurs sels d'addition avec un acide représentent de 0,001 à 10% en poids du poids total de la composition.

8. Composition selon la revendication 7, **caractérisée par le fait que** les 1-(4-aminophényl)-pyrrolidines de formule (I) et leurs sels d'addition avec un acide représentent de 0,01 à 8% en poids du poids total de la composition.

9. Composition selon l'une quelconque des revendications précédentes, **caractérisée par le fait que** les polymères cationiques à motifs récurrents de formule (III) sont des polymères constitués de motifs récurrents répondant à la formule (V) suivante: 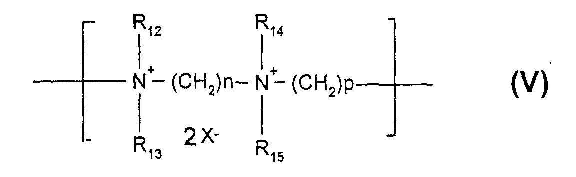 dans laquelle R₁₂, R₁₃, R₁₄ et R₁₅, identiques ou différents, désignent un radical alkyle ou hydroxyalkyle ayant de 1 à 4 atomes de carbone, n et p sont des nombres entiers variant de 2 à 20.

10. Composition selon la revendication 9, **caractérisée par le fait que** dans la formule (V), R₁₂, R₁₃, R₁₄ et R₁₅ représentent un radical méthyle, n = 3, p = 6 et X = Cl, [Polymère W].

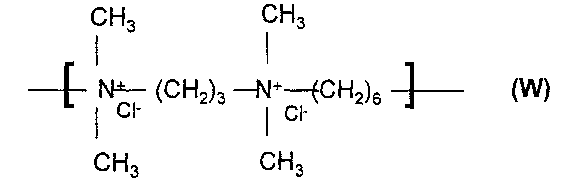

11. Composition selon la revendication 9, **caractérisée par le fait que** dans la formule (V), R₁₂, R₁₃, représentent un radical méthyle, R₁₄ et R₁₅ représentent un radical éthyle, n = p = 3 et X = Br, [Polymère U].

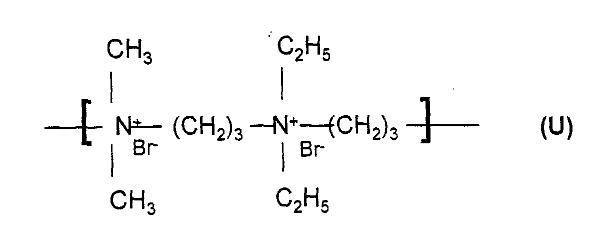

12. Composition selon l'une quelconque des revendications précédentes, **caractérisée par le fait que** dans la formule (IV) des polymères cationiques, p désigne un nombre entier variant de 1 à 6, D est nul ou représente un groupement―(CH₂)ᵣ―CO― dans lequel r désigne un nombre égal à 4 ou 7, et X- est un anion dérivé d'un acide minéral ou organique.

13. Composition selon la revendication 12, **caractérisée par le fait que** dans la formule (IV), p est égal à 3, D désigne la valeur zéro, X désigne un atome de chlore.

14. Composition selon l'une quelconque des revendications précédentes, **caractérisée par le fait que** les silicones aminées sont choisies parmi :
**(i)** l'amodiméthicone de formule (VI) suivante : 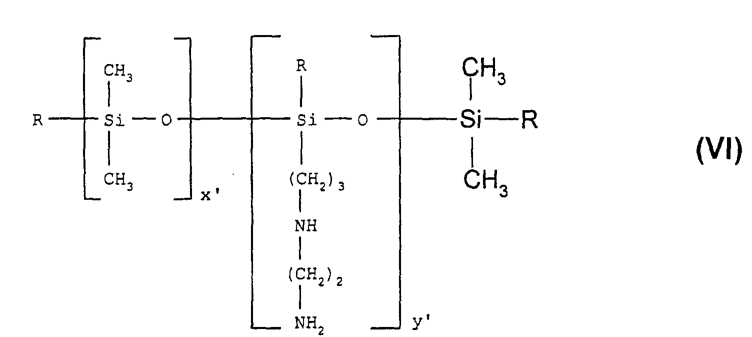 dans laquelle R désigne le radical CH₃ ou OH, et
x' et y' sont des nombres entiers dépendant du poids moléculaire, généralement tels que le poids moléculaire en nombre est compris entre 5 000 et 500 000 ;
**(ii)** les composés de formule (VII) suivante :
(R¹)ₐ (T)₃₋ₐ ―Si**[**OSi(T)₂**]**ₙ-**[**OSi(T)_{b}(R¹)_{2-b} **]**ₘ -OSi(T)₃₋ₐ - (R¹) ₐ (VII)
dans laquelle,
T est un atome d'hydrogène, ou un radical phényle, ou OH, ou alkyle en C₁-C₈,
a désigne zéro ou un nombre entier de 1 à 3,
b désigne zéro ou 1,
m et n sont des nombres tels que la somme (n + m) varie notamment de 1 à 2 000, n désignant un nombre de 0 à 1 999 et m désignant un nombre de 1 à 2000;
R¹ est un radical monovalent de formule -C_{q}H_{2q}L dans laquelle q est un nombre de 2 à 8 et L est un groupement aminé éventuellement quaternisé choisi parmi les groupements :
-N(R²)-CH₂-CH₂-N(R²)₂
-N(R²)₂
-N^{⊕}(R²)₃ Q⁻
-N^{⊕}(R²) (H)₂ Q⁻
-N^{⊕}(R²)₂H Q⁻
-N(R²)-CH₂-CH₂-N^{⊕}(R²)(H)₂ Q⁻,
dans lesquels R² désigne hydrogène, phényle, benzyle, ou un radical alkyle ayant de 1 à 20 atomes de carbone et Q⁻ représente un ion halogénure;
**(iii)** les composés de formule (IX) suivante : 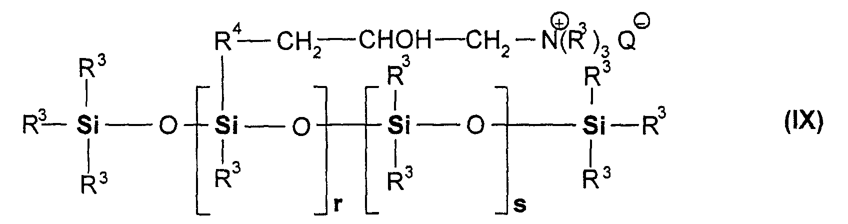 dans laquelle,
R³ représente un radical alkyle en C₁-C₁₈, ou alcényle en C₂-C₁₈,
R⁴ représente un radical alkylène en C₁-C₁₈ ou un radical alkylèneoxy divalent en C₁-C₁₈,
Q⁻ est un ion halogénure,
r représente une valeur statistique moyenne de 2 à 20;
s représente une valeur statistique moyenne de 20 à 200.
(iv) les composés de formule (X) suivante: 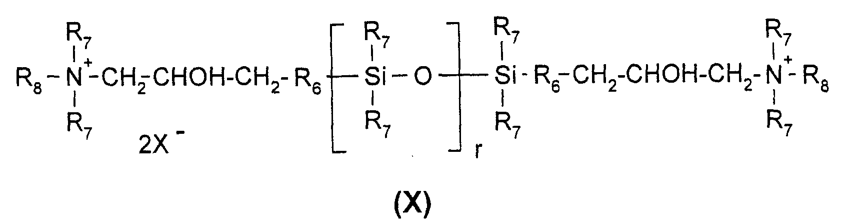 dans laquelle :
R₆ représente un radical alkylène en C₁-C₁₈ ou un radical alkylèneoxy divalent en C₁-C₁₈ relié au Si par une liaison SiC ;
les radicaux R₇, identiques ou différents, représentent un radical hydrocarboné monovalent ayant de 1 à 18 atomes de carbone, un radical alcényle en C₂-C₁₈ ou un cycle comprenant 5 ou 6 atomes de carbone ;
les radicaux R₈, identiques ou différents, représentent un atome d'hydrogène, un radical hydrocarboné monovalent ayant de 1 à 18 atomes de carbone, un radical alcényle en C₂-C₁₈, un radical -R₆-NHCOR₇;
r représente une valeur statistique moyenne de 2 à 200 ;
X- est un anion.

15. Composition selon la revendication 14, **caractérisée par le fait que** la silicone cationique de formule (VII) est la triméthylsilylamodiméthicone.

16. Composition selon la revendication 14, **caractérisée par le fait que** l'amodiméthicone est associée au chlorure de triméthylcétylammonium et au "trideceth-12".

17. Composition selon la revendication 15, **caractérisée par le fait que** la triméthylsilylamodiméthicone est associée à l' "octoxynol-40" et à l' "isolaureth-6".

18. Composition selon l'une quelconque des revendications précédentes,
**caractérisée par le fait que** le polymère cationique représente en poids de 0,01 à 10% du poids total de la composition.

19. Composition selon la revendication 18, **caractérisée par le fait que** le polymère cationique représente en poids de 0,05 à 5% du poids total de la composition.

20. Composition selon la revendication 19, **caractérisée par le fait que** le polymère cationique représente en poids de 0,1 à 3% du poids total de la composition.

21. Composition selon l'une quelconque des revendications précédentes,
**caractérisée par le fait qu'**elle contient au moins un coupleur.

22. Composition selon la revendication 21, **caractérisée par le fait que** les coupleurs sont choisis parmi les métaphénylènediamines, les métaaminophénols, les métadiphénols, les naphtols, les coupleurs hétérocycliques, et les sels d'addition de ces composés avec un acide.

23. Composition selon la revendication 22, **caractérisée par le fait que** les coupleurs sont choisis parmi le 2,4-diamino 1-(β-hydroxyéthyloxy) benzène, le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2-amino 4-(β-hydroxyéthylamino) 1-méthoxy benzène, le 1,3-diamino benzène, le 1,3-bis-(2,4-diaminophénoxy) propane, le sésamol, le 1-amino 2-méthoxy 4,5-méthylènedioxy benzène, l'α-naphtol, le 6-hydroxy indole, le 4-hydroxy indole, le 4-hydroxy N-méthyl indole, la 6-hydroxy indoline, la 2,6-dihydroxy 4-méthyl pyridine, le 1-H 3-méthyl pyrazole 5-one, le 1-phényl 3-méthyl pyrazole 5-one, la 2-amino 3-hydroxypyridine, le 3,6-diméthyl-pyrazolo-[3,2-c]-1,2,4-triazole, le 2,6-diméthyl-pyrazolo-[1,5-b]-1,2,4-triazole et leurs sels d'addition avec un acide.

24. Composition selon l'une quelconque des revendications 21-23,
**caractérisée par le fait que** les coupleurs sont présents dans des concentrations allant de 0,0001 à 15% en poids par rapport au poids total de la composition.

25. Composition selon l'une quelconque des revendications précédentes, **caractérisée par le fait qu'**elle contient au moins une base d'oxydation additionnelle dans la proportion de 0,0001 à 15% en poids par rapport au poids total de la composition.

26. Composition selon l'une quelconque des revendications précédentes, **caractérisée par le fait qu'**elle contient en outre au moins un colorant direct dans la proportion de 0,001 à 20% en poids par rapport au poids total de la composition.

27. Composition selon l'une quelconque des revendications précédentes, **caractérisée par le fait qu'**elle contient en outre au moins un agent réducteur ou antioxydant, dans des quantités allant de 0,05 à 1,5% en poids par rapport au poids total de la composition.

28. Composition prête à l'emploi pour la teinture d'oxydation des fibres kératiniques en particulier des fibres kératiniques humaines telles que les cheveux **caractérisée par le fait qu'**elle est obtenue par mélange d'une composition colorante telle que définie à l'une quelconque des revendications 1 à 27 et d'une composition oxydante contenant au moins un agent oxydant.

29. Composition selon la revendication 28, **caractérisée par le fait que** l'agent oxydant est choisi parmi le peroxyde d'hydrogène, le peroxyde d'urée, les bromates ou ferricyanures de métaux alcalins, les persels, les enzymes d'oxydoréduction telles que les laccases, les peroxydases et les oxydoréductases à 2 électrons, le cas échéant en présence de leur donneur ou cofacteur respectif.

30. Composition selon la revendication 29, **caractérisée par le fait que** l'agent oxydant est le peroxyde d'hydrogène.

31. Composition selon la revendication 30, **caractérisée par le fait que** l'agent oxydant est une solution d'eau oxygénée dont le titre varie de 1 à 40 volumes.

32. Composition selon l'une quelconque des revendications précédentes,
**caractérisée par le fait qu'**elle possède un pH allant de 3 à 12.

33. Composition selon la revendication 28, **caractérisée par le fait que** la composition colorante et/ou la composition oxydante contient au moins un tensioactif choisi parmi les tensioactifs anioniques, cationiques, non ioniques ou amphotères dans la proportion de 0,1 à 20% en poids par rapport au poids total de la composition.

34. Procédé de teinture d'oxydation des fibres kératiniques, et en particulier des fibres kératiniques humaines telles que les cheveux, consistant à appliquer sur les fibres une composition colorante contenant, dans un milieu approprié pour la teinture, au moins une 1-(4-aminophényl)-pyrrolidine de formule (I) telle que définie à l'une quelconque des revendications 1 à 8, la couleur étant révélée à pH alcalin, neutre ou acide à l'aide d'une composition oxydante qui est mélangée juste au moment de l'emploi à la composition colorante ou qui est appliquée séquentiellement sans rinçage intermédiaire, au moins un polymère cationique tel que défini à l'une quelconque des revendications 1, 9-20 étant présent dans la composition colorante et/ou oxydante.

35. Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins deux compartiments, dont l'un d'entre eux contient au moins une 1-(4-aminophényl)-pyrrolidine de formule (I) telle que définie à l'une quelconque des revendications 1 à 8, et un deuxième compartiment contenant au moins un agent oxydant, au moins un polymère cationique tel que défini à l'une quelconque des revendications 1, 9-20 étant présent dans le premier compartiment et/ou dans le second compartiment.

36. Dispositif à plusieurs compartiments ou " Kit " pour la teinture d'oxydation des fibres kératiniques et en particulier des fibres kératiniques humaines telles que les cheveux, **caractérisé par le fait qu'**il comporte au moins un compartiment contenant au moins une 1-(4-aminophényl)-pyrrolidine de formule (I) telle que définie à l'une quelconque des revendications 1 à 8, au moins un compartiment contenant au moins un polymère cationique tel que défini à l'une quelconque des revendications 1, 9-20, et au moins un autre compartiment contenant au moins un agent oxydant.
